# EUROPEAN PATENT SPECIFICATION

(11) **EP 3 255 426 B1**
(45) Date of publication and mention of the grant of the patent: **16.03.2022**
(21) Application number: 17172034.5
(22) Date of filing: 19.05.2017
(51) Int. Cl.: G01N 33/02

(54) **TOXICANT ASSAYS FOR CONSUMABLE PRODUCTS**
GIFTSTOFFTESTS FÜR VERBRAUCHSARTIKEL
DOSAGES DE SUBSTANCES TOXIQUES POUR PRODUITS CONSOMMABLES

(30) Priority: 08.06.2016 US 201662347129 P
(43) Date of publication of application: 13.12.2017
(73) Proprietor: Vitargent (International) Biotechnology Limited, Shatin, N.T. (HK)
(72) Inventor: CHEN, Xueping, Shatin, N.T. (HK); CHEN, Zixiang, Shatin, N.T. (HK); TAO, Wai Leung, Shatin, N.T. (HK)
(74) Representative: J A Kemp LLP

(56) References cited:
- EP-A2- 2 368 430
- US-A1- 2011 212 194
- YAU-HUNG CHEN ET AL: "Evaluation of the structureactivity relationship of flavonoids as antioxidants and toxicants of zebrafish larvae", FOOD CHEMISTRY, ELSEVIER LTD, NL, vol. 134, no. 2, 15 February 2012 (2012-02-15), pages 717-724, XP028417755, ISSN: 0308-8146, DOI: 10.1016/J.FOODCHEM.2012.02.166 [retrieved on 2012-03-06]
- KANG MIN-CHEOL ET AL: "Protective effect of marine algae phlorotannins against AAPH-induced oxidative stress in zebrafish embryo", FOOD CHEMISTRY, ELSEVIER LTD, NL, vol. 138, no. 2, 12 November 2012 (2012-11-12), pages 950-955, XP028977456, ISSN: 0308-8146, DOI: 10.1016/J.FOODCHEM.2012.11.005
- RANDRIAMAMPIANINA L ET AL: "Marked toxicity of Albizia bernieri extracts on embryo-larval development in the medaka fish (Oryzias latipes)", TOXICON, ELMSFORD, NY, US, vol. 64, 5 March 2013 (2013-03-05), pages 29-35, XP002706329, ISSN: 0041-0101, DOI: 10.1016/J.TOXICON.2012.12.012 [retrieved on 2012-12-31]
- M. CARO ET AL: "Zebrafish dives into food research: effectiveness assessment of bioactive compounds", FOOD & FUNCTION, vol. 7, no. 6, 7 April 2016 (2016-04-07), pages 2615-2623, XP055397899, GB ISSN: 2042-6496, DOI: 10.1039/C6FO00046K

## Description

### 1. FIELD OF INVENTION

The invention relates to a method of determining an overall toxicity in a consumable product. Particularly, the invention relates to methods using a teleost embryo to determine the overall toxicity in a consumable product.

### 2. BACKGROUND

With the rise in modernization and globalization, consumable products, such as foods and beverages, can go through many processes in which potentially toxic chemicals can enter the products before reaching the consumer. The US Environmental Protection Agency tracks or regulates more than 100,000 chemicals (Substance Registry Services Fact Sheet, available at ofmpub.epa.gov/sor_internet/registry/substreg/educationalresources), and the toxicity of many of these chemicals has not been well studied, especially their total biological toxicity when combined with other chemicals. Ensuring the safety of consumable products is a great challenge to the modern testing industry given the sheer number of potentially toxic chemicals and chemical combinations that can find their way into consumable products.

To date, toxicity testing of consumables still largely relies on chemical-specific tests, especially chemical analysis. For example, a review article introduces determination of pesticide residues in food matrices using QuEChERs methodology (Angelika Wilkowsk and Marek Biziuk, Food Chemistry 125 (2011), pp. 803-812). While chemical-specific tests can be sensitive and precise, they can fail to detect unknown toxicants that are not intended to be specifically tested; this can allow unanticipated toxicants to go undetected. Even in cases where the chemical composition of a sample is known in detail, its effective toxicity cannot necessarily be reliably predicted due to the lack of knowledge concerning the effects of chemical mixtures. Practical experience with studies has shown that chemical-specific measurements identify true toxicity in unknown samples only about 20% of the time, which means up to 80% toxicants are unidentified.

Thus, new methods for determining whether a toxicant is present in a consumable product are needed. US 2013/152222 relates to transgenic fishes and their use in, inter alia, detecting estrogenic and anti-estrogenic compounds, monitoring estrogen-like activity in the environment, and elucidating liver regeneration. However, there is a need to develop a bioassay to detect toxicants in a sample. Randriamampianina et al. (Toxicon. 2013 Mar 15;64:29-35) discloses marked toxicity of Albizia bernieri extracts on embryo-larval development in the medaka fish (Oryzias latipes). EP 2368430 A2 discloses transgenic fish and uses thereof.

### 3. SUMMARY

The invention is defined by the claims. Further aspects and preferred embodiments are defined in the dependent claims. Any aspects, embodiments and examples of the present disclosure which do not fall under the scope of the appended claims do not form part of the invention and are merely provided for illustrative purposes.

The disclosure provides methods of determining whether a toxicant is present in a consumable product, such as a food or beverage, which comprise contacting a teleost embryo with an extract from a sample of a consumable product and determining whether the extract exerts a toxicity effect on the embryo, where a toxicity effect on the embryo is indicative of the presence of a toxicant in the consumable product. Exemplary consumable products that can be tested using the methods of the disclosure are described in Section 4.2. The extract may comprise an organic solvent extract as recited (*e.g.*, an acetonitrile extract), which is optionally dehydrated and/or delipidated, wherein delipidation is only for a sample that contains lipid or rich-lipid. For non or low lipid containing samples, no delipidation is needed. Methods for preparing an extract from a consumable product for toxicant testing are described in Section 4.4 below.

The testing methods of the disclosure comprise determining whether the extract exerts a toxicity effect on teleost embryos, such as an acute effect (*e*.*g*., malformation or death) or a specific effect (*e*.*g*., estrogen activity disruption). The testing methods may comprise contacting a teleost embryo with an organic solvent extract that is obtainable or obtained by a process as described in Section 4.3 below. Exemplary methods of determining whether a toxicant is present in a consumable product are described in Sections 4.4 below. The teleost may be a medaka or a zebrafish embryo (as described in Sections 4.4.1-4.4.2), and may be transgenic. Acute and specific toxicity effects that can be determined using the methods of the disclosure are described in Sections 4.4.3 and 4.4.4, respectively. Exemplary acute toxicity effects include mortality, and malformation, and exemplary specific toxicity effects include estrogen activity disruption, androgen activity disruption, xenobiotic effect, cardiotoxicity effect, and hepatotoxicity effect.

The sample testing methods provided by the disclosure can be used, for example, to evaluate the total biological toxicity effects of extracts from consumable products. The methods of the disclosure can be applied in a high throughput manner to test large numbers of samples, providing, for example, a means to determine the biological safety of a large number of consumable products.

### 4. DETAILED DESCRIPTION

The technical information set out below may in some respects go beyond the disclosure of the invention, which is defined exclusively by the appended claims. The additional technical information is provided to place the actual invention in a broader technical context and to illustrate possible related technical developments. Such additional technical information which does not fall within the scope of the appended claims, is not part of the invention.

### 4.1. Overview

The invention creates a biological assay method for determining a toxicity profile in a consumable product sample. Significantly different from a chemical assay directed to detection of a specific toxicant(s) but not unspecified toxicants, the biological assay of the invention obtains an overall toxicity profile in the sample that can be used as index of toxicity of a sample. In contrast to the chemical assay, no toxic substance is added to the sample during the sample pretreatment process used in the method of the invention and agents used in the method will not react with the sample and change sample toxicity.

The disclosure provides methods of determining whether a toxicant is present in a consumable product. The methods comprise contacting a teleost embryo with an extract from a sample of the consumable product and determining whether the extract exerts a toxicity effect on the embryo, where a toxicity effect on the embryo indicates the presence of a toxicant in the consumable product. Consumable products which can be tested using the methods of the disclosure include, but are not limited to, feed, human foods, pet foods, and beverages. Advantageously, the methods of the disclosure can be used to monitor the total biological toxicity of a consumable, in contrast to chemical specific tests which generally detect the presence of only one type of toxicant or one group of toxicants. Methods of determining overall toxicity in a consumable product are illustrated in Section 4.2. Exemplary consumable products that can be tested using the methods of the disclosure are described in Section 4.3. Processes for preparing extracts from samples of consumable products are described in detail in Section 4.4, and methods of determining whether a toxicant is present in the consumable product extract are described in Section 4.5.

### 4.2. Method of determining overall toxicity in a consumable product

The invention provides a method of determining an overall toxicity in a consumable product, comprising:
a) combining a polar organic solvent and the consumable product to obtain a polar organic extract with an overall toxicity;
b) contacting a teleost embryo with the organic extract of a); and
c) determining whether the extract exerts a toxicity effect on the embryo;
   wherein a toxicity effect on the embryo shows an overall toxicity of the consumable product; wherein the consumable product is a lipid-containing consumable product, and the polar organic extract of step a) is further delipidated with a non-polar solvent;
   wherein if the sample is a low lipid containing sample it is not delipidated with a non-polar solvent; wherein the polar organic solvent is acetonitrile, ethanol, propanol, isopropanol, or a mixture containing two or more solvents thereof;
   wherein the teleost is fish of the *Oryzias* genus, the *Danio* genus or the *Pimephales* genus; and wherein the volume of the polar organic solvent combined with the consumable sample to form a mixture is 1 to 5 times the volume of the sample, when the sample is a liquid; or the volume of the polar organic solvent combined with the consumable sample to form a mixture is 1 to 5 times the weight of the sample, when the sample is a solid or semi-solid.

The polar organic solvent disclosed herein is used to extract most toxicants in a consumable product. The polar organic solvent includes acetonitrile, ethanol, propanol, isopropanol, and a mixture containing two or more solvents thereof. The volume of organic solvent combined with the sample to form the mixture is 1 to 5 times the volume or weight of the sample. That is, when the sample is a liquid, the volume of the polar organic solvent may be about 1 to about 5 times the volume of the sample; when the sample is a solid or semi-solid, the volume of the polar organic solvent is about 1 to about 5 times the weight of the sample. Preferably, the volume of organic solvent combined with the sample to form the mixture can be about 1 to about 4 times, about 1 to about 3 times, about 1 to about 2 times or about 1 times the volume or weight of the sample.

In one embodiment, before the above step a), water is added to the consumable product when the product is water unsaturated.

In another aspect, there is provided a method of determining an overall toxicity in a lipid-containing consumable product, wherein the extract of step a) is further delipidated.

The lipid-containing consumable product may contain a lipid that causes the polar solvent extract cannot be completely dried. Preferably, the lipid is in an amount of higher than 5% (w/w). More preferably, the lipid is in an amount of higher than 10% (w/w), 20% (w/w), 30% (w/w), 40% (w/w), 50% (w/w), 60% (w/w), 70% (w/w), 80% (w/w) or 90% (w/w). The delipidation step may be performed by adding a non-polar solvent to the organic solvent extract. Examples of the non-polar solvent include, but are not limited to, pentane, cyclopentane, hexane, cyclohexane, benzene, toluene, chloroform, diethyl ether, dichloromethane and a mixture containing two or more solvents thereof.

The method of the invention can be used to determine an overall toxicity in an edible oil such as vegetable and nut oils, as well as lard and other animal fats. Zebrafish embryos may be used for toxicity testing, which offers an accurate and quick approach to assess the safety of edible oils. For example, a testing method, combining a simple and easy edible oil extraction method, with a zebrafish embryo-based acute toxicity test to measure the acute toxicity (such as LC50) of the extract is established.

In one embodiment, the chemical contaminants of the consumable product are determined by a chemical analysis method to establish the correlation between the teleost embryo toxicity test and chemical contaminants.

### 4.3. Consumable products

The consumable product can be a product that is intended for human consumption (*e*.*g*., a food or a beverage) or animal consumption (*e*.*g*., pet foods such as a dog food or a cat food or livestock feed such as pig, goat or chicken feed). The consumable product may be intended for human consumption. The consumable product may be intended for animal consumption. Consumable products that can be tested using the methods of the disclosure include foods, beverages, and ingredients used to make a food or a beverage. As used herein, the term "food" encompasses food for human consumption and feed for animal consumption (including for consumption by livestock and for consumption by pets). Foods that can be tested include edible oil, ready to eat foods (*e.g*., cooked foods, canned foods, foods packaged in single or multiple serving packages, or animal feed), dairy products, meats, vegetables, fruits, infant formula, and dietary supplements derived from dairy, meat, vegetables, fruits, or a combination thereof (*e*.*g*., protein powders made from dairy, meat, or vegetables). As used herein, the term "edible oil" refers to a food substance, other than a dairy product, of whatever origin, source or composition that is manufactured for human consumption wholly or in part from a fat or oil other than that of milk. Edible oils that can be tested include plant-derived edible oils or fats, animal-derived edible oils or fats and synthetic oils and fats.

Exemplary dairy products that can be tested using the methods of the disclosure include milk (*e*.*g*., fresh milk, condensed milk, or powdered milk), buttermilk, cream, ice cream, yogurt, butter, cheese, and protein powders (*e*.*g*., whey concentrate, whey isolate, casein concentrate, or casein isolate).

Exemplary meats that can be tested using the methods of the disclosure include beef, pork (*e.g*., ham), lamb, goat, seafood (*e.g*., fish, shrimp, lobster, crab, clams, oysters, octopus, or squid), and poultry (*e.g*., chicken, duck, turkey, or goose). Meat can be fresh, cooked, or processed (*e.g*., dried or salted).

Exemplary fruits that can be tested using the methods of the disclosure include bananas, mangos, citrus fruits (*e*.*g*., oranges, lemons, limes, or grapefruit), apples, pears, peaches, plums, pineapples, berries (*e*.*g*., strawberries, blackberries, raspberries, or cranberries), lychees, and grapes. Exemplary vegetables include cabbages, turnips, radishes, carrots, lettuces, beans, peas, potatoes, eggplants, squashes, and onions. Fruits and vegetables can be fresh, cooked, or processed (*e*.*g*., jellies, jams, potato chips).

Exemplary beverages that can be tested using the methods of the disclosure include soft drinks (*e*.*g*., beverages containing milk, tea, coffee, juice, or sugar, or a combination thereof), and alcoholic drinks (*e*.*g*., beer, wine, cider, or spirits).

Exemplary food ingredients that can be tested using the methods of the disclosure include table sugar, brown sugar, corn syrup, carboxymethylcellulose, maltodextrin, demineralized whey powder, lactose, and oligosaccharides.

Exemplary edible oils that can be tested using the methods of the disclosure include olive oil, palm oil, soybean oil, canola oil (rapeseed oil), corn oil, peanut oil, corn oil, cottonseed oil, rice bran oil, coconut oil, peanut oil, sesame oil, pumpkin oil, sunflower oil, walnut oil, mustard oil, other vegetable oils and fats, butter, lard, beef tallow, other animal-based oils and fats and margarine.

The foregoing exemplary categories are not intended to be limiting, and inclusion of a consumable product in one category does not exclude its inclusion in another. For example, milk can be considered a dairy product and a beverage.

### 4.4. Consumable product extracts

Extracts that can be used in the toxicity testing methods of the disclosure are prepared from samples of consumable products, such as those described previously in Section 4.3. The sample can be an entire product (*e*.*g*., the entire contents of a single serving package of food) or a portion thereof. The sample can be, but is not necessarily, homogenized prior to extraction. Methods for homogenizing samples are known in the art, and include grinding (*e*.*g*., using a mortar and pestle), blending (*e*.*g*., with a blender), and sonication. Extract preparation from some consumable products may not benefit from a homogenization step, such as homogeneous dairy products or beverages, while others, such as meats or ready-to-eat foods such as rice dishes may benefit from a homogenization step prior to extraction.

Extracts that can be used in the toxicity testing methods of the disclosure are preferably polar organic solvent extracts. The term "polar organic solvent" when used in connection with the term "polar organic solvent extract" refers to the particular organic solvent or mixture of polar organic solvents used to extract compounds from a sample of a consumable product and does not necessarily refer to the solvent in which the extract may be dissolved in at any given time. For example, an extract prepared by extracting compounds from a sample using acetonitrile remains an acetonitrile extract even in instances in which the extract is processed to remove the acetonitrile following extraction. Thus, for example, an acetonitrile extract that has been dried to remove the acetonitrile and subsequently redissolved or resuspended in another solvent remains an acetonitrile extract even though the extract in its present state contains another solvent other than acetonitrile.

Exemplary organic solvent extracts include acetonitrile extracts, ethanol extracts, propanol extracts, isopropanol extracts, and a mixture containing two or more solvents thereof. The organic solvent extract may be an acetonitrile extract. The polar organic solvent extract may be an ethanol extract. Processes for making organic solvent extracts and reagents that can be used to make polar organic solvent extracts are described in Section 4.4.1.

Polar organic solvent extracts can optionally be dehydrated and/or can optionally be delipidated. Processes for dehydrating an organic solvent extract and reagents that can be used to dehydrate an organic solvent extract are described in Section 4.4.2. Processes for delipidating an organic solvent extract and reagents the can be used to delipidate an organic solvent extract are described in Section 4.4.3.

### 4.4.1. Extract preparation

Polar organic solvent extracts can be obtained by a process in which the first step comprises forming a mixture comprising a sample of the consumable product (*e.g*., a homogenized sample), a polar organic solvent and, optionally, a first salt and/or a sugar. In some embodiments, the mixture comprises the sample, the polar organic solvent, and a first salt. In other embodiments, the mixture comprises the sample, the polar organic solvent and a sugar. In other embodiments, the mixture comprises the sample, the polar organic solvent, a first salt and a sugar.

Without being bound by theory, it is believed that the first salt and/or sugar can promote the formation of at least two liquid phases in the mixture, one of which is enriched in the organic solvent relative to the other phase(s), and can promote the extraction of toxicants from the mixture into the phase enriched in the polar organic solvent. For example, acetonitrile is water miscible, and the addition of a salt or a sugar to a mixture containing acetonitrile and water can help to establish two liquid phases, one of which is enriched in acetonitrile. When using a polar organic solvent that is immiscible with a liquid contained in the sample (*e*.*g*., when forming a mixture of a water immiscible organic solvent such as toluene and a water containing food sample), the addition of salt may not be necessary to establish two liquid phases, although use of a first salt and/or sugar may aid in the extraction of toxicants from the mixture to the polar organic solvent.

The volume of the polar organic solvent can be selected or varied based upon the amount and/or nature of the sample (*e*.*g*., the consistency of the sample). The volume of polar organic solvent combined with the sample to form the mixture is 1 to 5 times the volume or weight of the sample; that is, when the sample is a liquid, the volume of the polar organic solvent is about 1 to about 5 times the volume of the sample; when the sample is a solid or semi-solid, the volume of the polar organic solvent is about 1 to about 5 times the weight of the sample. (*e*.*g*., about 1 to about 5 times, about 1 to about 4 times, about 1 to about 3 times, about 1 to about 2 times), about 1.5 to about 5 times the volume or weight of the sample (*e*.*g*., about 1.5 to about 4 times, about 1.5 times to about 3 times), about 2 times to about 5 times (*e*.*g*., about 2 to about 5 times, about 2 to about 4 times, about 2 times to about 3 times), or about 2 times to about 4 times the volume or weight of the sample). The volume of the polar organic solvent (*e*.*g*., acetonitrile) may be at least 1.5 times the volume or weight of the sample. Higher polar organic solvent to sample ratios can in some instances allow for higher amounts of extracted toxicants in contrast to lower polar organic solvent to sample ratios; however, higher polar organic solvent to sample ratios require more reagents (*e.g*., solvents and salts) and result in more dilute extracts.

First salts that can be used in the extraction process include sodium chloride, magnesium sulfate, sodium sulfate, calcium sulfate, calcium chloride, magnesium chloride, sodium acetate, ammonium acetate, anhydrous sodium sulfate, anhydrous magnesium sulfate, anhydrous sodium sulfate, anhydrous calcium sulfate, anhydrous calcium chloride, anhydrous calcium sulfate, and combinations thereof. The use of an anhydrous salt can help to saturate water solubility and "squeeze" the toxicants into organic solvent layer. In the context of this disclosure, and unless required otherwise by context, a salt that is not specifically identified as being in hydrated or anhydrous form encompasses both hydrated and anhydrous forms of the salt. For example, "calcium chloride" encompasses anhydrous and hydrated forms of calcium chloride (*i.e*., CaCl₂(H₂O)ₓ, where x = 0, 1, 2, 4, or 6).

In some embodiments, the first salt comprises sodium chloride, magnesium sulfate, sodium sulfate, calcium sulfate, calcium chloride, magnesium chloride, sodium acetate, ammonium acetate, or a combination thereof. In some embodiments, the salt comprises sodium chloride.

In other embodiments, the first salt comprises a combination of (i) sodium chloride, magnesium sulfate, calcium chloride, magnesium chloride, sodium acetate, ammonium acetate, or a combination thereof, and (ii) anhydrous sodium sulfate, anhydrous magnesium sulfate, anhydrous sodium sulfate, anhydrous calcium sulfate, anhydrous calcium chloride, anhydrous calcium sulfate, or a combination thereof. For example, the first salt can be a combination of sodium chloride and anhydrous sodium sulfate.

Sugars that can be used in the extraction process include monosaccharides and disaccharides, such as glucose, xylose, arabinose, fructose, maltose, sucrose and mixtures thereof. In some embodiments, the sugar comprises sucrose.

The first salt and/or sugar can be combined with the sample of the consumable product and the polar organic solvent to form the mixture, or the first salt and/or sugar can be added to a preformed mixture comprising the sample and the polar organic solvent. When the first salt and/or sugar is added to a preformed mixture, the mixture can be mixed before and/or after combining the mixture with the salt and/or sugar (*e*.*g*., by shaking the mixture, vortexing the mixture, sonicating the mixture, or vortexing and sonicating the mixture). The first salt and/or sugar can be added to the preformed mixture until the mixture is saturated with the first salt and/or sugar (*e*.*g*., as indicated by the observance of salt or sugar crystals within the mixture that do not dissolve).

Following formation of the mixture comprising the sample, the polar organic solvent and, optionally, a first salt and/or a sugar, the polar organic solvent extract can be obtained from the mixture by separating a phase containing the organic solvent from the mixture (*i.e*., a phase enriched in the organic solvent relative to the other phase(s)). Separation can comprise centrifuging the mixture to separate the phase containing the polar organic solvent from the mixture. Alternatively, the phases can be separated under the force of gravity, although separating a mixture under the force of gravity may take longer to complete compared to separating the mixture using centrifugation. The mixture may be mixed prior to the separation (*e*.*g*., by shaking the mixture, vortexing the mixture, sonicating the mixture, or vortexing and sonicating the mixture). Without being bound by theory, it is believed that mixing the mixture prior to separation can increase the yield of extracted toxicants.

Following separation, the polar organic solvent extract can be recovered, for example, by pipetting the phase containing the polar organic solvent away from the other phases, decanting the separated phases, or separating the phases using a separatory funnel (*e*.*g*., when centrifugation is not used to separate the phases). The recovered polar organic solvent extract can be further processed, for example, to remove water (*e.g*., as described in Section 4.3.2), to remove lipid (*e.g*., as described below in Section 4.3.3), to remove the polar organic solvent used for extraction, or any combination thereof. Further processing such as delipidation may not be performed. For example, delipidation steps may not be performed on polar organic solvent extracts made from samples comprising no lipids or low amounts of lipids.

The polar organic solvent used for extraction can be partially or completely removed by partially or completely by drying the polar organic solvent extract, for example under a stream of nitrogen or using a rotary evaporator. Alternatively, the polar organic solvent used for extraction can be removed by performing a solvent extraction on the organic solvent extract with another solvent. For example, when a solvent is used as the organic solvent for extracting compounds from the sample of the consumable product (*e.g*., a lipid rich product), a polar organic solvent (*e.g*., acetonitrile, dimethylformamide, or dimethyl sulfoxide) can be used to extract compounds (*e*.*g*., toxicants) and then hexane can be used to extract lipid from the polar organic extract. The polar organic solvent can then be removed from the extract if desired, for example by drying the extract under a stream of nitrogen or using a rotary evaporator.

Polar organic solvent extracts that have been dried can be redissolved or suspended in a second organic solvent. An organic solvent used for extraction can be removed at any time following extraction, for example, before or after subjecting the organic solvent extract to dehydration, or before or after subjecting the organic solvent extract to delipidation. The organic solvent used for extraction may be removed following dehydration and delipidation. In The second organic solvent may be a solvent that is appropriate for use in a toxicity assay as described in Section 4.4.4. Exemplary second organic solvents include methanol, dimethyl sulfoxide, and mixtures thereof.

### 4.4.2. Dehydration

A polar organic solvent extract, for example prepared by a process as described in Section 4.4.1, can be dehydrated to remove water that may be present in the polar organic solvent extract. For example, an acetonitrile extract prepared from a liquid containing food product can contain residual water. Polar organic solvent extracts containing water in addition to a polar organic solvent can be dehydrated by combining the extract with a second salt to form a mixture, and then separating a phase containing the organic solvent from the mixture. The second salt can be added to the polar organic solvent extract until the mixture is saturated in the second salt (*e.g*., as indicated by the presence of salt crystals on the surface of the mixture or within the mixture). After formation of the mixture, the mixture can be mixed (*e*.*g*., by shaking the mixture, vortexing the mixture, sonicating the mixture, or vortexing and sonicating the mixture).

The second salt can be an anhydrous salt, such as anhydrous sodium sulfate, anhydrous magnesium sulfate, anhydrous calcium sulfate, anhydrous calcium chloride, or a combination thereof. Other salts that can absorb water can also be used. In some embodiments, the second salt comprises anhydrous sodium sulfate.

Following formation of the mixture comprising the second salt and the polar organic solvent extract, a phase containing the polar organic solvent can be separated from the mixture. Separation can comprise centrifuging the mixture to separate the phase containing the polar organic solvent from the mixture. Alternatively, the phases can be separated under the force of gravity. Following separation, the polar organic solvent extract can be recovered, for example, by pipetting the phase containing the polar organic solvent away from the other phases, decanting the separated phases, or separating the phases using a separatory funnel (*e*.*g*., when centrifugation is not used to separate the phases).

### 4.4.3. Delipidation

A polar organic solvent extract, for example prepared by a process as described in Section 4.3.1 or 4.3.2, can be delipidated to remove lipids that may be present in the polar organic solvent extract. Removal of lipids can be accomplished, for example, by washing the polar organic solvent extract at least once (*e.g*., once, twice, or three times) with a non-polar solvent. For example, C₅-Cs alkanes (*e*.*g*., *n*-pentane, *n*-hexane, *n*-heptane, or *n*-octane) can be used. The solvent used for delipidation may comprise hexane. As used herein, "hexane" refers to *n*-hexane.

Delipidation can be performed on a polar organic solvent extract prepared as described in Section 4.4.1 or 4.4.2 without any intervening processing steps. Delipidation can also be performed on a polar organic solvent extract prepared as described in Section 4.4.1 or 4.4.2 that has undergone further processing steps, for example, partial solvent removal. The volume of non-polar solvent (*e*.*g*., hexane) used in each wash can be, for example, approximately one half to two thirds of the volume of the organic solvent extract (*e*.*g*., about 50%, about 55%, about 60%, or about 65%).

As an alternative to performing a polar organic solvent extraction followed by a wash with a nonpolar solvent as described above, a delipidated organic solvent extract can be obtained by using hexane as the solvent to extract compounds from the consumable product and then subjecting the hexane extract to solvent extraction using a polar organic solvent such as acetonitrile as described above in Section 4.3.1. This may be used, for example, to make delipidated extracts from lipid rich samples.

Delipidation is only for a sample that contains lipid or lipid rich. For non or low lipid containing samples, no delipidation is needed. It has been found that if a delipidation step is not performed on lipid containing samples, the organic extract cannot be completely dried, resulting to varying final volume between samples, even when processed identically. Thus, delipidation of lipid containing samples can help to standardize the volume of organic solvent extract obtained when processing multiple samples. For samples containing no lipid or low amounts of lipid (*e.g*., low fat or fat free fruits and vegetables), it may be desirable to omit a delipidation step.

### 4.4.4. Exemplary extraction protocol

The following protocol is an exemplary protocol for preparing a dehydrated and delipidated acetonitrile extract from a consumable product such as a food.
1) combine a sample of the consumable product with an amount of acetonitrile to form a mixture, and optionally mix the mixture;
2) add sodium chloride to the mixture until saturation, and optionally mix the mixture;
3) separate a phase containing acetonitrile from the mixture (*e*.*g*., by centrifugation) to obtain an acetonitrile extract;
4) add anhydrous sodium sulfate to the acetonitrile extract until saturation to form a second mixture, and optionally mix the second mixture;
5) separate a phase containing acetonitrile from the second mixture (*e*.*g*., by centrifugation) to obtain a dehydrated acetonitrile extract;
6) wash the dehydrated acetonitrile extract with hexane, and optionally repeat once to obtain a dehydrated and delipidated acetonitrile extract; and
7) optionally remove the acetonitrile from the dehydrated and delipidated acetonitrile extract and redissolve the dehydrated and delipidated acetonitrile extract in methanol.

The following protocol is an exemplary protocol for obtaining a dehydrated and delipidated hexane extract from a consumable product such as a lipid rich food.
1) combine a sample of the consumable product with an amount of hexane to form a mixture, and optionally mix the mixture;
2) add sodium chloride to the mixture until saturation, and optionally mix the mixture;
3) separate a phase containing hexane from the mixture (*e*.*g*., by centrifugation) to obtain a hexane extract;
4) add anhydrous sodium sulfate to the hexane extract until saturation to form a second mixture, and optionally mix the second mixture;
5) separate a phase containing hexane from the second mixture (*e*.*g*., by centrifugation) to obtain a dehydrated hexane extract;
6) perform a solvent extraction on the dehydrated hexane extract using acetonitrile (keeping the acetonitrile layer) to obtain a dehydrated and delipidated hexane extract; and
7) optionally remove the acetonitrile from the dehydrated and delipidated hexane extract and redissolve the dehydrated and delipidated hexane extract in methanol.

The organic solvent extract produced using the foregoing exemplary extraction protocol is referred to as a hexane extract throughout the protocol even though the protocol includes a second solvent extraction step using acetonitrile because hexane is the solvent used to perform the initial solvent extraction on the sample of the consumable product (see Section 4.4).

### 4.5. Testing for a toxicity effect

Teleost embryos are an effective *in vivo* model system to screen/identify the biological effects, *e.g.,* toxicity effects, of a test sample, and the adverse effects identified using fish (*e.g*., zebrafish and medaka fish) embryos is predictable to that of human beings. Fish embryos are not defined as protected animals under European legislation can be used as animal alternatives (Directive 2010/63/EU; Halder et al., 2010, Integrated Environmental Assessment Management. 6:484-491).

The screening assays of the disclosure entail contacting a teleost embryo with an extract from a sample of the consumable product and determining whether the extract exerts a toxicity effect on the embryo.

The teleost embryos that can be used in a screening assay of the disclosure can be of various freshwater, brackish water, or saltwater (marine water) species of fish, including, without limitation, fish of the *Oryzias* genus, the *Danio* genus and the *Pimephales* genus. Fish in the *Oryzias* genus belong to the *Adrianichthyidae* family and include, for example, *Oryzias melastigma* (alternative name *Oryzias dancena*) (marine or brackish medaka), *Oryzias latipes* (Japanese medaka), *Oryzias celebensis*, *Oryzias marmoratus*, *Oryzias matanensis*, *Oryzias nigrimas* (black buntingi), *Oryzias orthognathus* (buntingi), and *Oryzias profundicola.* Fish in the *Danio* genus belong to the *Cyprinidae* family and include, for example, *Danio rerio* (zebrafish), *Danio albolineatus*, *Danio abolineatus, Danio choprae, Danio dangila*, *Danio erythromicron, Danio feegradei*, *Danio kerri, Danio kyathit*, *Danio margaritatus*, *Danio meghalayensis*, *Danio nigrofasciatus*, and *Danio roseus.* Fish in the *Pimephales* genus belong to the Cyprinidae family and include *Pimephales notatus* (bluntnose minnow), *Pimephales promelas* (fathead minnow), *Pimephales tenellus* (slim minnow), and *Pimephales vigilax* (bullhead minnow). The fish embryos may be Japanese or brackish medaka fish, zebrafish or fathead minnow embryos. Particular advantages of brackish medaka fish and zebrafish are described in Sections 4.5.1 and 4.5.2, respectively.

The toxicity effect can be an acute toxicity effect (as described in Section 4.5.3) or a specific toxicity effect (as described in Section 4.5.4).

The fish embryos can be transgenic or non-transgenic. Non-transgenic fish can be used, for example, for detection of an acute toxicity effect in extracts from consumable products, *e*.*g*., toxicity, as described in Section 4.5.3. Transgenic fish embryos are particularly useful when screening for a specific effect, *e.g*., for detection of estrogenic compounds and anti-estrogenic compounds in extracts from consumable products as described in Section 4.5.4.1 below.

The screening assays can be performed in a high throughput or semi high throughput manner, *e*.*g*., in multiwell plates (e.g., 24, 96 or 384 well plates), and/or with positive and/or negative controls (*e*.*g*., medium only as a negative control and an agent known to exert a toxicity effect in the particular assay as a positive control). Each extract in an assay can be tested in duplicate or triplicate. The assays can be performed using multiple dilutions of each extract.

### 4.5.1. Medaka fish

The brackish medaka fish (*Oryzias melastigma*) is native to coastal waters and fresh waters in Pakistan, India, Burma and Thailand (Naruse, 1996, Fish Biol. L. Medaka 8:1-9), and thrives in waters of varying salinity ranging from 0 parts per thousand (ppt) to as high as 35 ppt. Additionally, this brackish medaka fish has a number advantages for transgenic development, including: (1) small size (2-3 cm for adult fish); (2) relatively short generation time (2-3 months); (3) dimorphic sex (*e*.*g*., females have a flat distal surface of the anal fin, while that of males is convex due to separated longer fin rays); (4) high prolific capacity to reproduce; (5) translucent eggs and larvae (up to 15 days post fertilization), which facilitates the positioning of DNA microinjection needles and observation of internal organs; and (6) adaptable to various transgenic techniques used to produce transgenic fish of other *Oryzias* species (*e.g*., *Oryzias latipes*).

Regarding the highly prolific capacity of the brackish medaka fish to reproduce, spawning of this fish can be induced all year round, and each pair of female and male fish can produce 20-30 eggs daily for up to several months under indoor maintained conditions (*e*.*g*., 28±1°C with a constant light cycle of 14 h-light/8 h-dark and fed with commercial hormone-free flake food and brine shrimp (*Artemia salina*)). Eggs usually hatch in 11 to 15 days at 28±1°C.

The two medaka species of *Oryzias melastigma* and *Oryzias latipes* share high morphological, physiology, and genomic similarity, and while *Oryzias latipes* was first used to produce transgenic fish, the transgenic techniques were readily adapted to the brackish medaka *Oryzias melastigma* (Chen et al., 2008, Ectoxicol. Environ. Saf 71:200-208; Chen et al., 2009, Comp. Biochem. Physiol. C. Toxicol. Pharmacol. 149:647-655).

Medaka can be bred to be see-through (see, *e.g*., U.S. Patent No. 6,737,559), further facilitating screening assays, particularly those involving detecting reporter expression or activity levels.

### 4.5.2. Zebrafish

Research has shown that zebrafish are a good model to predict toxicity of human drugs. There are close physiological and genetic similarities between zebrafish and mammalian species, and researchers have conducted systematic evaluations of zebrafish toxicity end points using large numbers of pharmacologically relevant compounds.

As an experimental tool, zebrafish have an array of advantages such as optical transparency, high fecundity, and quick, external development. Changes to morphology and modulations in gene and protein expression can be easily assayed through the use of fluorescent proteins. The relatively small physical size allows for multiple zebrafish to fit into a multiwell plate, making the scaling of experiments an easy transition. Also, the relatively cheaper costs associated with fish husbandry, coupled with the frequency of progeny that zebrafish can achieve, are other reasons that make this organism an attractive tool for screening assays.

### 4.5.3. Acute toxicity effect

The consumable product extracts of the disclosure can be measured for acute toxicity effects such as mortality and malformation on a whole organism level.

Taking zebrafish as an example, the zebrafish embryo toxicity test is based on a 48 h exposure of newly fertilized eggs in a static or semi-static system. Various endpoints such as coagulation of eggs and embryos, failure to develop somites, lack of heart-beat as well as non-detachment of the tail from the yolk are indicative of toxicity. These endpoints can be recorded after, *e.g*., 24, 48, 72 and 96 hr and used for the calculation of an LC₅₀ value of a consumable product extract. Analogous endpoints can be measured in Japanese medaka fish and in fathead minnows (see Braunbeck & Lammer, 2006, Background Paper on Fish Embryo Toxicity Assays, available from www.oecd.org/chemicalsafety/testing/36817242.pdf).

### 4.5.4. Specific toxicity effect

The consumable product extracts of the disclosure can also be assayed for specific effects, *i.e*., effects on particular tissue, organ, or hormone system. Assays of particular interest include those for cardiotoxicity, ototoxicity, seizure liability, endocrine disruption, gastrointestinal motility, hepatotoxicity, skin pigmentation alterations, muscle toxicity, pancreatic toxicity, carcinogenesis, neurotoxicity, and renal toxicity (see, *e.g*., Sarvaiya et al., 2014, Veterinary Clinical Science 2(3):31-38, Peterson and MacRae, 2011, Annu. Rev. Pharmacol. Toxicol. 52:433-53, Eimon and Rubenstein, 2009, Expert Opin. Drug Metab. Toxicol. 5(4):393-401, and references cited therein for assay details).

In certain aspects, specific toxicity effect can be measured by detecting alterations in gene expression a result of exposure of a teleost embryo to a consumer product extract. To facilitate observation of alterations in gene expression, a transgenic teleost embryo in which a regulatory sequence of interest (*e*.*g*., an inducible promoter) is operably linked to a reporter sequence can be used. The regulatory sequence can be from the fish species under study or a different fish species, as long as it behaves appropriately in the fish species being assayed. Alterations in expression of the reporter following exposure of a consumer product extract as compared to a (negative and/or positive) control can be detected and/or measured.

Suitable reporter sequences will be evident to those of skill in the art. For example, a suitable reporter protein can include fluorescent proteins and enzymes detectable by a histochemical method. The reporter sequences can be introduced into teleost genomes in constructs containing appropriate exogenous regulatory elements (*e*.*g*., promoter and 3' untranslated regions, for example as described in U.S. Patent No. 9,043,995) or can be knocked into an endogenous genetic locus (for example using the methodology described in Kimura et al., 2014, Scientific Reports 4:6545, doi:10.1038).

Fluorescent proteins are well known in the art. Examples of fluorescent proteins include, without limitation, a green fluorescent protein (GFP), an enhanced green fluorescent protein (EGFP), a red fluorescent protein (CFP and Red FP, RFP), a blue fluorescent protein (BFP), a yellow fluorescent protein (YFP), and fluorescent variants of these proteins. The heterologous fluorescent gene (the term gene in this context refers to any coding sequence, with or without control sequences) may be, for example, a gene encoding DsRed2, ZsGreen1, and ZsYellow1. The heterologous fluorescent gene may encode any naturally occurring or variant marker proteins, including green fluorescent protein (GFP), enhanced green fluorescent protein (eGFP), yellow fluorescent protein (YFP), enhanced yellow fluorescent protein (eYFP), blue fluorescent protein (BFP), enhanced blue fluorescent protein (eBFP), cyan fluorescent protein (CFP), and enhanced cyan fluorescent protein (eCFP).

Enzymes that are detectable by histochemical methods are also well known in the art. Examples of enzymes include, without limitation, luciferase, horseradish peroxidase, β-galactosidase, β-glucuronidase, alkaline phosphatase, chloramphenicol acetyl transferase, and alcohol dehydrogenase. The enzyme may be luciferase. The term "luciferase" is intended to denote all the proteins which catalyze or initiate a bioluminescent reaction in the presence of a substrate called luciferin. The luciferase may be from any organism or system that generates bioluminescence (see, *e.g*., U.S. Patent No. 6,152,358). For example, the luciferase may be from *Renilla* (U.S. Patent Nos. 5,418,155 and 5,292,658), from *Photinus pyralis* or from *Luciola cruciata* (U.S. Patent No. 4,968,613).

Techniques to detect protein reporters, either directly (*e*.*g*., by measuring the amount of reporter mRNA) or indirectly (*e*.*g*., by measuring the amount and/or activity of the reporter protein) are conventional. Many of these methodologies and analytical techniques can be found in such references as Current Protocols in Molecular Biology, F. M. Ausubel et al., eds., (a joint venture between Greene Publishing Associates, Inc. and John Wiley & Sons, Inc.), Enzyme Immunoassay, Maggio, ed. (CRC Press, Boca Raton, 1980); Laboratory Techniques in Biochemistry and Molecular Biology, T. S. Work and E. Work, eds. (Elsevier Science Publishers B. V., Amsterdam, 1985); Principles and Practice of Immunoassays, Price and Newman, eds. (Stockton Press, NY, 1991); and the like.

The amount and/or activity of a reporter expression product (*e.g*., a protein) may be measured. A fluorescent marker, such as eGFP, can be detected by detecting its fluorescence in the cell (*e.g*., in a brackish medaka fish or zebrafish embryo). For example, fluorescence can be observed under a fluorescence microscope and, if desired, can be quantitated. Reporters such as eGFP, which are directly detectable without requiring the addition of exogenous factors, are preferred for detecting or assessing gene expression during fish embryonic development. A transgenic fish embryo engineered to express fluorescent reporter under the control of a promoter of interest can provide a rapid real time *in vivo* system for analyzing spatial and temporal expression patterns.

### 4.5.4.1. Endocrine Disruptor Assays

Endocrine disruptors are chemicals that, at certain doses, can interfere with the endocrine (or hormone) system in mammals. These disruptions can cause cancerous tumors, birth defects, and other developmental disorders. Specifically, endocrine disruptors may be associated with the development of learning disabilities, severe attention deficit disorder, cognitive and brain development problems; deformations of the body; breast cancer, prostate cancer, thyroid and other cancers (see Gore et al., 2015, Endocrine Reviews 36(6):593-602. doi: 10.1210/er.2015-1093). One well known example of an endocrine disruptor is bisphenol A, a chemical commonly found in plastic bottles, plastic food containers, dental materials, and the linings of metal food and infant formula cans. Bisphenol A is associated with elevated rates of diabetes, mammary and prostate cancers, decreased sperm count, reproductive problems, early puberty, obesity, and neurological problems.

Endocrine disruptors can be evaluated in transgenic teleost embryos harboring a coding sequence for a marker protein operably linked to a promoter that is sensitive to disruptors of multiple endocrine systems. Because several hormones that operate in different endocrine system share common subunits, the use of a promoter from one of the common subunits permits interrogation of multiple hormone systems simultaneously. One example of such a subunit is the glycoprotein subunit α (*gsuα*), which encodes the shared α subunit of follicle stimulating hormone β, luteinizing hormone β, and thyroid-stimulating hormone (TSH) β. The *gsuα* promoter of zebrafish is an example of a promoter that can be operably linked to a coding sequence of a marker protein and used to detect endocrine disrupting chemicals (Cheng et al., 2014, Toxicology and Applied Pharmacology 278:78-84), and can be used to screen for the presence of endocrine disrupting chemicals in consumable products as described herein.

Many endocrine disruptors possess estrogenic, enhancing-estrogenic or anti-estrogenic properties. For the evaluation of the estrogenic, enhancing-estrogenic and anti-estrogenic properties of the consumable product extracts of the disclosure, the consumable product extracts can be assayed in teleost embryos harboring an estrogen responsive promoter operably linked to a coding sequence for a marker protein. The estrogen responsive promoter may be from a choriogenin gene of a medaka fish (*e.g*., *Oryzias melastigma* and *Oryzias latipes*), for example choriogenin H or choriogenin L. Choriogenin H and L are precursor proteins of the inner layer subunits of egg envelope (chorion) of teleost fish, and gene expression of both choriogenin H and choriogenin L are responsive to estrogenic substances (see, *e.g*., Yamaguchi et al., 2015, J Appl Toxicol. 35(7):752-8). The choriogenin H promoter may be used to assay the estrogen disruptor activity of a consumable product extract. The choriogenin H promoter has been shown to be a highly sensitive biomarker for monitoring estrogenic chemicals in the marine environment (Chen et al., 2008, Ecotoxicol Environ Saf. 71(1):200-8). Examples of choriogenin H promoter constructs suitable for use for assaying estrogenic activity of consumer product extracts are disclosed in U.S. Patent No. 9,043,995. The choriogenin L promoter may be used. The estrogen responsive promoter may be the brain aromatase B promoter (referred to as a cyp19a1b promoter in zebrafish). The zebrafish cyp19a1b gene exhibits exquisite sensitivity to estrogens and is a sensitive target for estrogen mimics, and has been successfully operably linked to a marker gene such as GFP in transgenic fish (see, *e.g*., Brion et al., 2012, PLoS ONE 7(5): e36069. doi:10.1371/journal.pone.0036069). The estrogen sensitive promoter may be a vitellogenin promoter (for example as described in Schreurs et al., 2004, Environmen. Sci. Technol. 34:4439-44).

Other endocrine disruptors possess androgenic, enhancing-androgenic or anti-androgenic properties. Androgenic, enhancing-androgenic and anti-androgenic properties of consumable product extracts can be evaluated in teleost embryos harboring an androgen responsive promoter operably linked to a coding sequence for a marker protein. The androgen responsive promoter may be the *G*. *aculeatus spiggin* promoter, which is responsive to androgens but exhibits no reactivity to, *inter alia*, estrogens and glucocorticoids (*see*, *e.g*., Sebillot et al., 2014, Environ. Sci. Technol. 48:10919-28).

Yet other endocrine disruptors possess thyroid-disrupting properties, *e*.*g*., they disrupt the hypothalamic-pituitary-thyroid (HPT) axis. Thyroid/HPT disrupting properties of consumable product extracts can be evaluated in teleost embryos harboring a thyroid hormone (TH) responsive promoter operably linked to a coding sequence for a marker protein. The thyroid responsive promoter may be the thyroid-stimulating hormone subunit β (TSHβ) promoter, which in contrast to other subunits is unique to TSH. Thyroid-stimulating hormone is part of a feedback loop involving TH and thyrotropin-releasing hormone (TRH). Specifically, when low levels of TH are present, TRH is secreted by the hypothalamus to stimulate the release of TSH by the pituitary, which in turn stimulates the thyroid to secrete TH, and the opposite feedback loop occurs when high levels of TH are present. The TSHβ promoter is a useful biomarker for the HPT axis. An example of a TSH β promoter that can be used is the zebrafish TSH β promoter (*see*, *e.g.*, Ji et al., 2012, Toxicology and Applied Pharmacology 262:149-155.

### 4.5.4.2. Xenobiotic Assays

Xenobiotics are foreign chemical substances present within an organism. Xenobiotics may be grouped as antioxidants, carcinogens, drugs, environmental pollutants, food additives, hydrocarbons, and pesticides. Pollutants such as dioxins and polychlorinated biphenyls are considered xenobiotics. The body removes xenobiotics by xenobiotic metabolism. This consists of the deactivation and the excretion of xenobiotics, and happens mostly in the liver, by way of reactions catalyzed by the hepatic microsomal cytochrome P450 enzyme system.

For the evaluation of the xenobiotic properties of the consumable product extracts of the disclosure, the consumable product extracts can be assayed in teleost embryos harboring a xenobiotic responsive promoter operably linked to a coding sequence for a marker protein. The promoter may be a cytochrome P450 promoter, *e*.*g*., the zebrafish P450 1A (Cyp1a) promoter such as described in Boon and Gong, 2013, PLOS ONE 8(5):e64334.

Xenobiotic properties of food product samples and food product extracts of the disclosure can also be evaluated using an *in vivo* ethoxyresorufin-O-deethylase (EROD) activity assay using 7-ethoxyresorufin as substrate, for example as described in Liu et al., 2014, Environmental Toxicology 31(2):201-10.

### 4.5.4.3. Hepatotoxicity assays

Zebrafish have been studied as models of drug-induced hepatotoxicity. The transparency of zebrafish for several days post-fertilization enables *in* vivo visual observation of internal organs including liver. Zebrafish complete primary liver morphogenesis by 48 hours post-fertilization (HPF). When exposed to a hepatotoxicant, changes to liver morphology can be evaluated visually (Hill et al., 2012, Drug Metabolism Reviews 44(1): 127-140). Researchers have developed various endpoints that can be studied to evaluate hepatotoxicity: liver degeneration, changes in size and shape of the liver, and yolk sac retention (see He et al., 2013, Journal of Pharmacological and Toxicological Methods 67:25-32). These parameters can be assayed in zebrafish to evaluate the hepatotoxicity of a consumable product extract of the disclosure, and analogous parameters can be used to assay the hepatotoxicity of a consumable product extract in a different fish such as medaka.

### 4.5.4.4. Cardiotoxicity assays

Teleost embryos provide an ideal model system for investigating cardiotoxicity because their transparency and uncovered hearts make them easily observable. Taking zebrafish as an example, the heart consists of a ventricle and an atrium and these develop rapidly. Heart tube and heartbeat are observed at 24 hours post fertilization (hpf), and then tube looping, chamber formation, and blood circulation are completed by 72 hpf.

It is possible to assay consumable product extracts for cardiotoxicity by evaluating parameters such as heart rate, rhythmicity (*e*.*g*., atrioventricular block (AV block), arrhythmia); circulation, and morphology (*e*.*g*., pericardial edema; hemorrhage, heart chamber swelling) in teleost embryos.

The heart-specific promoter BMP4 can be used to drive expression of a marker gene that allows heart morphology to be observed. The erythrocyte-specific promoter *gata*1 can be used to drive expression of a marker gene, allowing the blood circulation rate to be observed (see Wu et al., 2013, Toxicol. Sci. 136(2):402-412, and references cited therein).

These parameters can be assayed in zebrafish or medaka to evaluate the cardiotoxicity of a consumable product extract of the disclosure.

### EXAMPLES

### 5.1. Example 1: Chicken breast extract preparation

Chicken breast produced by farms A, B and C were extracted for acute toxicity and estrogenic activity testing. Chicken breast samples were mechanically homogenized. The homogenized meat were aliquoted and mixed with 1:1.5 (w/v) acetonitrile. After vortexing and sonication, sodium chloride was added until saturation. Samples were centrifuged at 5,000 x g for 10 minutes and the supernatant was collected. Anhydrous sodium sulfate was added to the supernatant until saturation. The supernatant was separated and dried under nitrogen gas flow until about 5 ml remained, and then twice washed using 3 ml hexane. The sample was then dried under nitrogen gas flow and redissolved using 200 µl of absolute methanol and stored at -20°C until testing.

### 5.2. Example 2: Acute toxicity testing of chicken breast extract

Extracts prepared as described in Example 1 were tested for acute toxicity using zebrafish (*Danio rerio*) AB strain embryos. Chicken breast extracts were diluted into zebrafish embryo culture medium at 0.25, 0.50, 1.00, 2.00 and 4.00 µl/ml. Zebrafish AB strain embryos of 4-128 cell stages were exposed to extract dilutions in a 96-well plate at 1 embryo per well. Each concentration was tested with 20 embryos. Zebrafish embryo culture medium and 3.7 mg/L dichloroaniline were included as negative and positive controls, respectively. After 48 hr exposure at 26°C, zebrafish embryos were observed under a stereomicroscope and fish embryos that were coagulated, tail not detached, and having no heart beat were marked as dead. Mortality rate for each concentration was calculated as the acute toxicity endpoint. The mortality rate for the negative control was 0% and for the positive control was 65%. Table 1 shows the acute toxicity test results. Of the three chicken breast extracts, farm A sample extract showed the highest acute toxicity while farm B sample extract showed the lowest toxicity to zebrafish embryos.

| **Table 1: Acute toxicity of chicken breast extracts from farms A, B and C** | | | | | | |
|---|---|---|---|---|---|---|
| Tested sample nominal concentration | | 19 g/L | 41 g/L | 90 g/L | 197 g/L | 433 g/L |
| Mortality rate of each sample | Farm A | 0% | 20% | 100% | 100% | 100% |
| | Farm B | 5% | 0% | 10% | 10% | 5% |
| | Farm C | 0% | 0% | 35% | 30% | 90% |

### 5.3. Example 3: Estrogenic activity of chicken breast extract

Extracts prepared as described in Example 1 were tested for estrogenic activity using choriogenin H - eGFP transgenic medaka (*Oryzias melastigma*) eleutheroembryos generated as described in Example 1 of U.S. Patent No. 9,043,995. Chicken breast extracts were diluted into medaka (*Oryzias melastigma*) embryo culture medium (instant ocean salt dissolved in deionized water to make 0.2% salinity) at 2.5 µl/ml. 17β-estradiol was also tested at 1.0, 2.0, 5.0 and 10.0 µg/L as positive controls. Culture medium was tested as negative control. Each concentration contained 3 replicates with each replicate containing 8 eleutheroembryos. After 24-hr exposure at 26°C, eleutheroembryos were observed under green fluorescence microscope and imaged from ventral side using the same imaging setting. Negative control and extracts of samples from farms A and B did not induce observable green fluorescence in the eleutheroembryo livers. The extract of the sample from farm C induced observable green fluorescence in the eleutheroembryo livers.

### 5.4. Example 4: Milk powder extract preparation

Brands A, B and C of formula milk powder for 1-3 year old children were extracted for acute toxicity and estrogenic activity testing. Milk powder was reconstituted with water and mixed with 1:1.5 (v/v) acetonitrile. After vortexing and sonication, sodium chloride was added until saturation and then centrifuged to separate the phases. Anhydrous sodium sulfate was added to the supernatant until saturation. The supernatant was separated and dried under nitrogen gas flow until about 5 ml remained, and then twice washed using 3 ml hexane. The sample was then dried under nitrogen gas flow and re-dissolved using 200 µl of absolute methanol and stored at -20°C until testing.

### 5.5. Example 5: Acute toxicity testing of milk powder extract

The acute toxicity of milk powder extracts prepared as described in Example 4 were tested for acute toxicity using zebrafish (*Danio rerio*) AB strain embryos. Milk powder extracts were diluted into zebrafish embryo culture medium at 0.33, 0.50, 0.76, 1.74 and 4.00 µl/ml. Zebrafish AB strain embryos of 4-128 cell stages were exposed to extract dilutions in a 96-well plate with 1 embryo per well. Each concentration was tested with 20 embryos. Zebrafish embryo culture medium and 3.7 mg/L 3, 4-dichloroaniline were included as negative and positive controls, respectively. After 48-hr exposure at 26°C, zebrafish embryos were observed under stereomicroscope and fish embryos that were coagulated, tail not detached and having no heart beat were classified as dead. Mortality rate of each concentration was calculated as the acute toxicity endpoint. Mortality rate for negative control was 5% and for positive control was 60%. Table 2 shows the test results. Of the three formula milk extracts, the extract of brand A was the most toxic and the extract of brand C extract was the least toxic to zebrafish embryos.

| **Table 2: Acute toxicity of 3 brands of formula milk extract** | | | | | | |
|---|---|---|---|---|---|---|
| Tested sample nominal concentration | | 21g/L | 47 g/L | 103 g/L | 227 g/L | 500 g/L |
| Mortality rate of each sample | Brand A | 5% | 0% | 5% | 100% | 100% |
| | Brand B | 0% | 10% | 0% | 0% | 100% |
| | Brand C | 0% | 5% | 0% | 15% | 15% |

### 5.6. Example 6: Estrogenic activity of milk powder extract

The estrogenic activity of milk powder extracts prepared as described in Example 4 were tested using choriogenin H - EGFP transgenic medaka (*Oryzias melastigma*) eleutheroembryos. Milk powder extracts were diluted into medaka (*Oryzias melastigms*) embryo culture medium (instant ocean salt dissolved in deionized water to make 0.2% salinity) at 2.5 µl/ml. 17β-estradiol was also tested at 1.0, 2.0, 5.0 and 10.0 µg/L as positive controls. Culture medium was tested as negative control. Each concentration contained 3 replicates with each replicate contained 8 eleutheroembryos. After 24-hr exposure at 26°C, eleutheroembryos were observed under green fluorescence microscope and imaged from ventral side using the same imaging setting. Extracts of samples from brand A and brand C did not induce observable green fluorescence in the eleutheroembryo livers, while the extract of formula milk of brand B did induce observable green fluorescence in the eleutheroembryo livers. Table 3 shows milk powder estrogenic activity data. The estrogen equivalent concentration in Table 3 means the estrogen activity of a sample equivalent to that of 17 beta-estradiol.

| **Table 3: Estrogen equivalent concentration of 3 baby milk formula extract** | | |
|---|---|---|
| Estrogen equivalent concentration of each sample | Brand A | Not detected |
| | Brand B | 20 ng/g |
| | Brand C | 30 ng/g |

### 5.7. Example 7: Assay for Cooking Oil

1 volume of cooking oil sample (lard or peanut oil) was mixed with 1:1.5 (v/v) of each of (1) acetone, (2) hexane (3) acetonitrile, (4) methanol, and (5) ethanol. After the resulting mixture was vortexed and centrifuged, the supernatant layers of mixtures (3)-(5) were collected and then dried under nitrogen gas flow. It was found that the solvents (1)-(2) in the mixtures cannot be separated from oil, so they both cannot be used in the assay. However, mixture (5) contains the most oil, whereas mixture (3) contains the least oil, so mixture (3) was selected to be used in the assay. Other solvents like toluene, ether, dichloromethane and chloroform were also used and are applicable to this step.

The solvent in mixture (3), acetonitrile, is water miscible and sodium chloride can help to separate water from the mixture. Magnesium sulfate, calcium chloride, magnesium chloride, sodium acetate, ammonium acetate and sucrose can also be used. After vortexing and sonication, sodium chloride was added to the supernatant (3) until saturation so that the water contained therein could be separated. The resulting sample was further subjected to vortexing and sonication and the supernatant was collected. Anhydrous sodium sulfate (or magnesium sulfate, sodium sulfate, calcium chloride or calcium sulfate) was added to the supernatant until saturation. The supernatant was separated and dried under nitrogen gas flow until about 5 ml remained, and then twice washed using 3 ml hexane. The resulting supernatant was then dried under nitrogen gas flow and redissolved using 200 µl of absolute methanol and stored at -20°C until testing.

The estrogenic activity and acute toxicity testing of the cooking oil samples were conducted according to the methods described in the above sections 5.5 (Example 5) and 5.6 (Example 6) and the results show that it can be identified whether a toxicant is present in the cooking oil. The acute toxicity data of lard extract and peanut oil extract are shown in Table 4 and Table 5 below.

| **Table 4: Acute toxicity of 3 lard extract** | | | | | | |
|---|---|---|---|---|---|---|
| Tested sample nominal concentration | | 19 mL/L | 41 mL/L | 90 mL/L | 197 mL/L | 433 mL/L |
| Mortality rate of each sample | Brand A | 0% | 0% | 5% | 0% | 205 |
| | Brand B | 5% | 40% | 100% | 100% | 100% |
| | Brand C | 100% | 100% | 100% | 100% | 100% |

| | | | | | | |
|---|---|---|---|---|---|---|
| Brand A: known well refined lard from good origin Brand B: known tainted (gutter) Brand C: known unrefined lard | | | | | | |

| **Table 5: Acute toxicity of 3 peanut oil extract** | | | | | | |
|---|---|---|---|---|---|---|
| Tested sample nominal concentration | | 19 mL/L | 41 mL/L | 90 mL/L | 197 mL/L | 433 mL/L |
| Mortality rate of each sample | Brand A | 0% | 15% | 20% | 35% | 0% |
| | Brand B | 35% | 80% | 100% | 100% | 35% |
| | Brand C | 100% | 100% | 100% | 100% | 100% |

### 5.8. Example 8: Assay for Drink

Three soy milk sample of different brands (A, B and C) were extracted for toxicity testing. Acetonitrile was added to the sample at a ratio of 1.5:1 (v/v) to obtain an acetonitrile extract. The extract was dried under nitrogen gas flow and redissolved using methanol and stored at -20°C until testing.

The estrogenic activity and acute toxicity testing of three drink sample extract were conducted according to the methods described in the above sections 5.5 (Example 5) and 5.6 (Example 6) and the results show that it can be identified whether a toxicant is present in the soy milk. The acute toxicity data and the estrogen equivalent concentration of the soy milk extracts are shown in Table 6 and Table 7 below.

| **Table 6: Acute toxicity of 3 soy milk extract** | | | | | | |
|---|---|---|---|---|---|---|
| Tested sample nominal concentration | | 21g/L | 47 g/L | 103 g/L | 227 g/L | 500 g/L |
| Mortality rate of each sample | Brand A | 0% | 0% | 5% | 0% | 10% |
| | Brand B | 0% | 2% | 60% | 100% | 100% |
| | Brand C | 0% | 10% | 20% | 60% | 85% |

| **Table 7: Estrogen equivalent concentration of 3 soy milk extract** | | |
|---|---|---|
| Estrogen equivalent concentration of each sample | Brand A | Not detected |
| | Brand B | Not detected |
| | Brand C | 30 ng/mL |

### 5.9. Example 9: Lipid-containing Water-saturated Solid / Semi-solid Samples (e.g. yogurt)

The yogurt samples were homogenized. Acetonitrile was added to the sample in a ratio of 1.5:1 (v/w) to obtain an acetonitrile extract. Anhydrous Na₂SO₄ was added the acetonitrile extracts to remove water. The resulting extract was dried under nitrogen gas flow and re-dissolved using methanol and stored at -20°C until testing.

The estrogenic activity and acute toxicity testing of three yogurt sample extract were conducted according to the methods described in the above sections 5.5 (Example 5) and 5.6 (Example 6) and the results show that it can be identified whether a toxicant is present in the yogurt. The acute toxicity data and the estrogen equivalent concentration of the yogurt extracts are shown in Table 8 and Table 9 below.

| **Table 8: Acute toxicity of 3 yogurt extract** | | | | | | |
|---|---|---|---|---|---|---|
| Tested sample nominal concentration | | 21g/L | 47 g/L | 103 g/L | 227 g/L | 500 g/L |
| Mortality rate of each sample | Brand A | 0% | 25% | 59% | 100% | 10% |
| | Brand B | 0% | 10% | 30% | 60% | 100% |
| | Brand C | 0% | 10% | 0% | 30% | 65% |

| **Table 9: Estrogen equivalent concentration of 3 yogurt extract** | | |
|---|---|---|
| Estrogen equivalent concentration of each sample | Brand A | Not detected |
| | Brand B | Not detected |
| | Brand C | 14 ng/g |

### 5.10. Example 10: Lipid-containing Water-unsaturated Solid / Semi-solid Samples (e.g. wheat powder)

The wheat powder samples were homogenized. Water was added to the resulting sample to form a mixture. Acetonitrile was added to the mixture at a ratio of 1.5:1 (v/v) to obtain an acetonitrile extract. NaCl was added the acetonitrile extract to saturate the water. Anhydrous Na₂SO₄ was added to the resulting extract to remove water and then hexane was added to the extract to remove lipid. The resulting extract was dried under nitrogen gas flow and redissolved using methanol and stored at -20°C until testing.

The estrogenic activity and acute toxicity testing of three wheat powder sample extract were conducted according to the methods described in the above sections 5.5 (Example 5) and 5.6 (Example 6) and the results show that it can be identified whether a toxicant is present in the wheat powder. The acute toxicity data and the estrogen equivalent concentration of the wheat powder extracts are shown in Table 10 and Table 11 below.

| **Table 10: Acute toxicity of 3 wheat powder extract** | | | | | | |
|---|---|---|---|---|---|---|
| Tested sample nominal concentration | | 21g/L | 47 g/L | 103 g/L | 227 g/L | 500 g/L |
| Mortality rate of each sample | Brand A | 0% | 5% | 0% | 0% | 10% |
| | Brand B | 0% | 0% | 10% | 20% | 30% |
| | Brand C | 0% | 5% | 15% | 30% | 65% |

| **Table 11: Estrogen equivalent concentration of 3 wheat powder extract** | | |
|---|---|---|
| Estrogen equivalent concentration of each sample | Brand A | Not detected |
| | Brand B | Not detected |
| | Brand C | Not detected |

### 5.11. Non-lipid-containing Water-saturated Solid / Semi-solid Samples (e.g. jam)

The jam samples were homogenized. Acetonitrile was added to the sample at a ratio of 1:1 (v/w) to obtain an acetonitrile extract. NaCl was added to the acetonitrile extract to saturate the water. Anhydrous Na2SO4 was added to the resulting extract to remove water. The resulting extract was dried under nitrogen gas flow and re-dissolved using methanol and stored at - 20°C until testing.

The estrogenic activity and acute toxicity testing of three jam sample extract were conducted according to the methods described in the above sections 5.5 (Example 5) and 5.6 (Example 6) and the results show that it can be identified whether a toxicant is present in the jam. The acute toxicity data and the estrogen equivalent concentration of the jam extracts are shown in Table 12 and Table 13 below.

| **Table 12: Acute toxicity of 3 jam extract** | | | | | | |
|---|---|---|---|---|---|---|
| Tested sample nominal concentration | | 21g/L | 47 g/L | 103 g/L | 227 g/L | 500 g/L |
| Mortality rate of each sample | Brand A | 0% | 5% | 0% | 70% | 100% |
| | Brand B | 0% | 0% | 0% | 20% | 70% |
| | Brand C | 0% | 50% | 100% | 100% | 100% |

| **Table 13: Estrogen equivalent concentration of 3 jam extract** | | |
|---|---|---|
| Estrogen equivalent concentration of each sample | Brand A | Not detected |
| | Brand B | Not detected |
| | Brand C | Not detected |

### 5.12. Non-lipid-containing Water-unsaturated Solid / Semi-solid Samples (e.g. animal feed)

The feed sample was homogenized. Acetonitrile was added to the sample at a ratio of 1.5:1 (v/w) to obtain an acetonitrile extract. NaCl was added to the acetonitrile extract to saturate the water. The resulting extract was added with anhydrous Na₂SO₄ to remove water. The resulting extract was dried under nitrogen gas flow and redissolved using methanol and stored at -20°C until testing.

The estrogenic activity and acute toxicity testing of three duck feed sample extract were conducted according to the methods described in the above sections 5.5 (Example 5) and 5.6 (Example 6) and the results show that it can be identified whether a toxicant is present in the duck feed. The acute toxicity data and the estrogen equivalent concentration of the duck feed extracts are shown in Table 14 and Table 15 below.

| **Table 14: Acute toxicity of 3 duck feed extract** | | | | | | |
|---|---|---|---|---|---|---|
| Tested sample nominal concentration | | 21g/L | 47 g/L | 103 g/L | 227 g/L | 500 g/L |
| Mortality rate of each sample | Brand A | 0% | 5% | 10% | 70% | 90% |
| | Brand B | 0% | 0% | 30% | 70% | 100% |
| | Brand C | 10% | 60% | 80% | 100% | 100% |

| **Table 15: Estrogen equivalent concentration of 3 duck feed extract** | | |
|---|---|---|
| Estrogen equivalent concentration of each sample | Brand A | Not detected |
| | Brand B | 21 ng/g |
| | Brand C | 91 ng/g |

### 5.13. Lipid-containing Liquid Samples (e.g. liquid milk)

Acetonitrile was added to a liquid milk sample at a ratio of 1.5:1 (v/v) to obtain an acetonitrile extract. NaCl was added to the acetonitrile extract to saturate the water. Anhydrous Na₂SO₄ was added to the resulting extract to remove water. The resulting extract was dried under nitrogen gas flow and redissolved using methanol and stored at -20°C until testing.

The estrogenic activity and acute toxicity testing of three liquid milk sample extract were conducted according to the methods described in the above sections 5.5 (Example 5) and 5.6 (Example 6) and the results show that it can be identified whether a toxicant is present in the liquid milk. The acute toxicity data and the estrogen equivalent concentration of the liquid milk extracts are shown in Table 16 and Table 17 below.

| **Table 16: Acute toxicity of 3 liquid milk extract** | | | | | | |
|---|---|---|---|---|---|---|
| Tested sample nominal concentration | | 43 mL/L | 94 mL/L | 207 mL/L | 455 mL/L | 1000 mL/L |
| Mortality rate of each sample | Brand A | 0% | 5% | 5% | 0% | 30% |
| | Brand B | 0% | 0% | 20% | 60% | 100% |
| | Brand C | 100% | 100% | 100% | 100% | 100% |

| **Table 17: Estrogen equivalent concentration of 3 liquid milk extract** | | |
|---|---|---|
| Estrogen equivalent concentration of each sample | Brand A | 3 ng/g |
| | Brand B | Not detected |
| | Brand C | Not detected |

### 5.14. Non-lipid-containing Liquid Samples (e.g. juice)

Acetonitrile was added to liquid milk sample at a ratio of 1.5:1 (v/v) to obtain an acetonitrile extract. NaCl was added to the acetonitrile extract to saturate the water. Anhydrous Na₂SO₄ was added to the resulting extract to remove water. The resulting extract was dried under nitrogen gas flow and re-dissolved using methanol and stored at -20°C until testing.

The estrogenic activity and acute toxicity testing of three grape juice sample extract were conducted according to the methods described in the above sections 5.5 (Example 5) and 5.6 (Example 6) and the results show that it can be identified whether a toxicant is present in the grape juice. The acute toxicity data and the estrogen equivalent concentration of the grape juice extracts are shown in Table 18 and Table 19 below.

| **Table 18: Acute toxicity of 3 grape juice extract** | | | | | | |
|---|---|---|---|---|---|---|
| Tested sample nominal concentration | | 43 mL/L | 94 mL/L | 207 mL/L | 455 mL/L | 1000 mL/L |
| Mortality rate of each | Brand A | 0% | 5% | 5% | 0% | 0% |
| | Brand B | 0% | 0% | 10% | 0% | 10% |
| sample | Brand C | 0% | 0% | 20% | 40% | 60% |

| **Table 19: Estrogen equivalent concentration of 3 grape juice extract** | | | | | | |
|---|---|---|---|---|---|---|
| Estrogen equivalent concentration of each sample | Brand A | Not detected | | | | |
| | Brand B | Not detected | | | | |
| | Brand C | Not detected | | | | |

### 5.15. Lard Sample

The acute toxicity (mortality at 48 hours of exposure) of extracts of 7 lard samples of known quality (state of refinement and purity) were tested with zebrafish embryos. Cutoff criteria for acceptance of toxicity data were set when the mortality rates of zebrafish embryos in the blank (culture medium) and solvent (0.4% methanol) controls were ≤10% and > 30% in the positive control (3.7 mg/L 3, 4-dichloroaniline). The LC₅₀ of the extract of each oil was calculated based on the embryo mortality rate versus dose response curve, and presented as the nominal concentration of the original oil. Table 20 shows that normally-produced lards (LN1 and LN2) exhibited low toxicity (LC₅₀ >173.3 mL/L), whilst unrefined lard (LT1 and LT2) exhibited high toxicity (LC₅₀ <14.3 mL/L) and the remaining lards, which were not well-refined, exhibited varying toxicity, with LC₅₀s between 14.3 mL/L and 173.3 mL/L.

**Table 20. Individual lard sample descriptors and respective acute toxicity values in zebrafish embryos**

| Sample Code ^{a} | Description | LC50 (mL/L) |
|---|---|---|
| LT1 | Tainted oil-Raw lard (unrefined) | <14.3 |
| LT2 | Tainted oil-Raw lard (unrefined) | <14.3 |
| LT3 | Tainted oil-Refined lard | 21.7 |
| LT4 | Tainted oil-Refined lard | 38.6 |
| LT5 | Tainted oil-Not proper refined oil | 48.3 |
| LN1 | Normal oil-Raw lard | >173.3 |
| LN2 | Normal lard-Raw lard | >173.3 |

| | | |
|---|---|---|
| ^{a} LT, Tainted lard; LN, Normal lard. | | |

To determine potential correlations between the zebrafish embryo toxicity test results and potential chemical contaminants, acetonitrile extracts of representative lards of low (LN1, LC50 >173.3 mL/L), moderate (LT5, LC50 = 48.3 mL/L) and high (LT1, LC50 <14.3 mL/L) toxicity, as classified by the bioassay, were subjected to non-targeted, high resolution LCMS analysis, with accompanying multivariate statistical analysis. The PCA analysis of m/z signals obtained for each lard extract tested, with clear separation based on the acute toxicity of the extract. Further to this, a total of 7 (positive ion mode) and 9 (negative ion mode) characteristic m/z signals (intensities between 293.2109 and 445.2786) were selected from the spectra of the high toxicity lard (LT1) for further identification (Table 21). In contrast to this, the signal intensities of LT5 and LN1 were approximately 10-100% and 0-20% that of LT1, respectively. Indeed, the low acute toxicity sample (LN1) was similar to the blank, indicating that the selected m/z features correlated positively with the zebrafish embryo toxicity test results. Furthermore, the identification results from the Progenesis QI software confirmed that most of the signals (i.e., all except m/z 354.2850 and 353.2299) matched the corresponding compounds (Table 21), identified by ChemSpider. In the negative ion mode, the possible compound candidates of m/z 311.2214, 309.52507 and 329.2319 were all lipid oxidation products (Table 22).

**Table 21. Chemical descriptor characteristics of selected m/z ions obtained from acetonitrile extracts of the most toxic lard (LT1) and their relative signal intensities in other tested lard samples of lower toxicity ^{a}.**

| **Information of characteristic signals** | | | | **Signal intensity** | **Relative signal intensity (%)** | | |
|---|---|---|---|---|---|---|---|
| m/z | major adduct | neutral mass (Da) | chemical formula | high toxicity LT1 | moderate toxicity LT5 | low toxicity LN1 | blank |
| Positive | | | | | | | |
| 408.2929 | M+Na | 385.3037 | C₁₉H₃₉N₅O₃ | 7.70E+05 | 25.7 ± 0.6 | 0.6 ± 0.1 | 0.4 ± 0.1 |
| 406.2747 | M+Na | 383.2853 | C₂₆H₄₀P | 6.29E+05 | 26.1 ± 2.1 | 0.9 ± 0.0 | 0.8 ± 0.1 |
| 341.2054 | M+Na | 318.2162 | C₁₆H₂₆N₆O | 5.07E+05 | 18.4 ± 0.9 | 5.5 ± 0.5 | 4.1 ± 0.2 |
| 393.2611 | M+Na | 370.2719 | C₂₁H₃₈O₅ | 3.73E+05 | 19.2 ± 0.4 | 6.2 ± 0.7 | 1.6 ± 0.2 |
| 357.2006 | M+Na | 334.2114 | C₁₆H₂₆N₆O₂ | 3.32E+05 | 41.5 ± 1.4 | 1.2 ± 0.5 | 0.3 ± 0.3 |
| 354.2850 | M+NH₄ | 336.2513 | No match | 4.25E+05 | 18.2 ± 0.6 | 5.8 ± 2.4 | 1.6 ± 0.0 |
| 353.2299 M+Na | 330.2408 | No match | 3.67E+05 | 14.8 ± 1.4 | 3.4 ± 0.9 | 4.4 ± 0.9 | |

| Negative | | | | | | | |
|---|---|---|---|---|---|---|---|
| 311.2214 M-H | 312.2287 | C₁₈H₃₂O₄ | 1.75E+06 | 101.1 ± 7.0 | 5.1 ± 0.1 | 4.1 ± 0.5 | |
| 299.2578 M-H | 300.2650 | C₁₈H₃₆O₃ | 1.22E+06 | 73.6 ± 2.1 | 16.6 ± 3.3 | 1.5 ± 0.3 | |
| 327.2162 M-H | 328.2235 | C₁₆H₃₀N₃O₄ | 1.14E+06 | 46.7 ± 5.1 | 5.0 ± 0.2 | 3.7 ± 0.1 | |
| 393.2240 M+Na-2H | 372.2493 | C₂₀H₃₆O₆ | 9.68E+05 | 10.4 ± 0.7 | 1.0 ± 0.1 | 0.9 ± 0.1 | |
| 309.2057 M-H | 310.2137 | C₁₈H₃₀O₄ | 9.03E+05 | 84.4 ± 2.6 | 4.6 ± 0.4 | 3.5 ± 0.4 | |
| 329.2319 M-H | 330.2399 | C₁₈H₃₄O₅ | 8.50E+05 | 37.2 ± 2.5 | 5.7 ± 0.3 | 6.1 ± 0.5 | |
| 393.2243 M+FA-H | 348.2260 | C₁₆H₃₂N₂O₆ | 6.32E+05 | 94.8 ± 5.1 | 8.2 ± 0.1 | 5.9 ± 0.7 | |
| 293.2109 M-H | 294.2181 | C₁₈H₃₀O₃ | 5.49E+05 | 71.2 ± 1.8 | 3.8 ± 0.6 | 1.2 ± 0.2 | |
| 445.2786 M+Na-2H | 424.3016 | C₁₈H₃₆N₁₀O₂ | 4.73E+05 | 29.0 ± 0.5 | 11.5 ± 0.9 | 0.3 ± 0.2 | |

| | | | | | | | |
|---|---|---|---|---|---|---|---|
| ^{a}The m/z order is dependent on the signal intensity of LT1. | | | | | | | |

**Table 22. Suggested structural identities of some m/z ions with strong correlation to the induction of acute toxicity of lard extracts in zebrafish embryos.**

| | | | | | |
|---|---|---|---|---|---|
| m/z | Major adduct | Neutral mass (Da) | Formula | Probable chemical compound | |
| | | | | Name | Structure |

| **Negative ion mode** | | | | | |
|---|---|---|---|---|---|
| 311.2214 | M-H | 312.2287 | C₁₈H₃₂O₄ | 8-{3-[(1E)-3-Hydroxy-1-octen-1 - yl]-2-oxiranyl}octanoic acid | 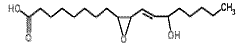 |
| 309.2057 | M-H | 310.2144 | C₁₈H₃₀O₄ | 9-Hydroxy-11-(3-pentyl-2-oxiranyl)-7,10-undecadienoic acid | 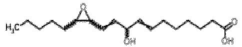 |
| 329.2319 | M-H | 330.2406 | C₁₈H₃₄O₅ | 9,10,13-Trihydroxy-11-octadecenoic acid | 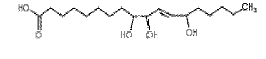 |
| | | | | | 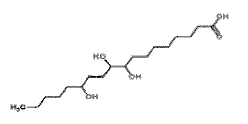 |
| | | | | | 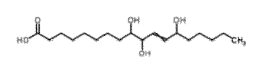 |
| | | | | Ethyl (6R)-6-hydroxy-6-{(2R,5R)-5-[(1S)-1-hydroxyhexyl]tetrahy dro-2-furanyl}hexanoate | 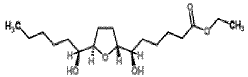 |

### 5.16. Peanut Oil Sample

The acute toxicity of 6 peanut oils from Mainland China and 3 peanut oils from Hong Kong were tested in this study. Table 23 shows that the acetonitrile extracts of these 9 peanut oils exerted varying acute toxicity in zebrafish embryo cultures, with LC₅₀s ranging from <14.3 mL/L to >173.3 mL/L. The extract of sample P1 showed the highest acute toxicity with an LC₅₀ <14.3 mL/L, whereas extracts of samples P7, P8 and P9 showed the lowest acute toxicity with LC₅₀s >173.3 mL/L. Extracts of the other 5 samples showed varying toxicity, with LC₅₀s between 14.3 mL/L and 173.3 mL/L. No obvious correlation between the description of state of purity of the oil and the respective LC₅₀ values was identified.

**Table 23. Individual peanut oil sample descriptors and respective acute toxicity values in zebrafish embryos**

| Sample Code | Description | Source | LC50 (ml/L) |
|---|---|---|---|
| P1 | Peanut oil | China market | <14.3 |
| P2 | Traditional method squeezed peanut oil | China market | 20.0 |
| P3 | Peanut oil | China market | 95.3 |
| P4 | Peanut oil | Hong Kong market | 104.2 |
| P5 | Five techniques processed peanut oil | China market | 133.0 |
| P6 | Peanut oil | China market | 140.0 |
| P7 | Refined peanut oil | China market | >173.3 |
| P8 | Pure peanut oil | Hong Kong market | >173.3 |
| P9 | Peanut oil | Hong Kong market | >173.3 |

The same procedure to that used with lard was used to determine if there was any correlation between the toxicity potential and chemical composition of peanut oils. The PCA scatter plot shows that oil extracts with varying toxicity can be clustered and separated based on the m/z signals from high resolution LC/MS analysis. However, there were no correlations in the characteristic chemical signals obtained from the most toxic peanut oil (P1, Table 24) and those obtained from the most toxic lard sample (LT1, Table 21). A total of 4 (positive ion mode) and 2 (negative ion mode) characteristic m/z signals were selected (Table 24), ranging between 165.0912 and 628.1956. The signal intensities of the low (P7) and moderately (P2) toxic peanut oils were approximately 10-90% and 0-20% that of P1 respectively, indeed m/z 299.1102, 195.1017 and 165.0912 all exhibited intensities <5% in the low and moderately toxic samples. The identification results from the Progenesis QI software, confirmed that most of the signals (except m/z 628.1956) matched the corresponding compounds listed in ChemSpider. However, unlike the lards, no lipid oxidation products were identified.

**Table 24. Chemical descriptor characteristics of selected m/z ions obtained from acetonitrile extracts of the most toxic peanut oil (P1) and their relative signal intensities in other tested peanut oil samples of lower toxicity ^{a}.**

| **Information of characteristic signals** | | | | **Signal intensity** | **Relative signal intensity (%)** | | |
|---|---|---|---|---|---|---|---|
| m/z | major adduct | neutral mass (Da) | chemical formula | **high toxicity** P1 | **moderate toxicity** P2 | **low toxicity** P7 | blank |
| **Positive** | | | | | | | |
| **397.1986** | **M+Na** | **374.2094** | C₁₈H₂₅FN₇O | 3.88E+05 | 52.3 ± 5.1 | 1.3 ± 0.0 | 0.0 ± 0.0 |
| **299.1102** | **M+Na** | **276.1212** | C₁₁H₁₅F₃N₄O | 3.85E+05 | 3.3 ± 0.3 | 4.7 ± 0.3 | 0.7 ± 0.1 |
| **195.1017** | **M+CH₃OH+H** | **162.0681** | C₁₀H₁₀O₂ | 2.50E+05 | 1.8 ± 0.5 | 1.7 ± 0.4 | 2.0 ± 1.0 |
| 628.1956 | M+NH₄ | 610.1617 | No match | 6.89E+06 | 87.9 ± 11.6 | 17.1 ± 1.0 | 2.8 ± 1.0 |

| **Negative** | | | | | | | |
|---|---|---|---|---|---|---|---|
| **317.1379** | **M-H** | **318.1452** | C₁₈H₂₂O₅ | 2.76E+05 | 8.2 ± 0.3 | 1.7 ± 0.2 | 0.0 ± 0.0 |
| **165.0912** | **M-H** | **166.0985** | C₆H₁₇NO₂P | 2.41E+05 | 0.0 ± 0.0 | 0.0 ± 0.0 | 0.2 ± 0.1 |

| | | | | | | | |
|---|---|---|---|---|---|---|---|
| ^{a} The m/z order is dependent on the signal intensity of P1. | | | | | | | |

## Claims

1. A method of determining an overall toxicity in a consumable product, comprising:
a) combining a polar organic solvent and the consumable product to obtain a polar organic extract with an overall toxicity;
b) contacting a teleost embryo with the organic extract of a); and
c) determining whether the extract exerts a toxicity effect on the embryo;
wherein a toxicity effect on the embryo shows an overall toxicity of the consumable product; wherein the consumable product is a lipid-containing consumable product, and the polar organic extract of step a) is further delipidated with a non-polar solvent;
wherein if the sample is a low lipid containing sample it is not delipidated with a non-polar solvent;
wherein the polar organic solvent is acetonitrile, ethanol, propanol, isopropanol, or a mixture containing two or more solvents thereof;
wherein the teleost is fish of the *Oryzias* genus, the *Danio* genus or the *Pimephales* genus; and wherein the volume of the polar organic solvent combined with the consumable sample to form a mixture is 1 to 5 times the volume of the sample, when the sample is a liquid; or the volume of the polar organic solvent combined with the consumable sample to form a mixture is 1 to 5 times the weight of the sample, when the sample is a solid or semi-solid.

2. The method of Claim 1, wherein the consumable product is a food, edible oil or feed, optionally wherein the food is a packaged food, a dairy product, meat, vegetables, fruits, infant formula or a beverage.

3. The method of Claim 1, wherein the polar organic solvent is acetonitrile.

4. The method of any one of the preceding claims, wherein
the volume of the polar organic solvent combined with the consumable sample to form a mixture is 1 to 3 times the volume of the sample, when the sample is a liquid; or the volume of the polar organic solvent combined with the consumable sample to form a mixture is 1 to 3 times the weight of the sample, when the sample is a solid or semi-solid.

5. The method of any one of the preceding claims, wherein before the above step a), water is added to the consumable product when the product is water unsaturated.

6. The method of any one of the preceding claims, optionally wherein
(i) the lipid-containing consumable product contains a lipid that prevents the polar organic extract from being completely dried;
(ii) the lipid-containing consumable product contains a lipid higher than 5% (w/w); or
(iii) the non-polar solvent is pentane, cyclopentane, hexane, cyclohexane, benzene, toluene, chloroform, diethyl ether, dichloromethane or a mixture containing two or more the solvents thereof.

7. The method of any one of the preceding claims, wherein in step a), a first salt and/or a sugar is added to obtain the polar organic extract.

8. The method of Claim 7, wherein the first salt is sodium chloride, magnesium sulfate, sodium sulfate, calcium sulfate, calcium chloride, magnesium chloride, sodium acetate, ammonium acetate, anhydrous sodium sulfate, anhydrous magnesium sulfate, anhydrous sodium sulfate, anhydrous calcium sulfate, anhydrous calcium chloride, anhydrous calcium sulfate, or a combination thereof.

9. The method of Claim 7, wherein the sugar is sucrose.

10. The method of any one of the preceding claims, wherein the method further comprises a step of adding a second salt to the organic solvent extract from step (a) to form a mixture.

11. The method of Claim 10, wherein the second salt is an anhydrous sodium sulfate, anhydrous magnesium sulfate, anhydrous calcium sulfate, anhydrous calcium chloride, or a combination thereof.

12. The method of any one of the preceding claims, wherein the process further comprises a step of homogenizing the sample prior to step (a); drying the organic solvent extract and redissolving the organic solvent extract in a second organic solvent; or recovering the organic solvent extract.

13. The method of any one of the preceding claims, wherein the teleost embryo is a zebrafish embryo or medaka embryo.

14. The method of any one of the preceding claims, wherein the chemical contaminants of the consumable product are determined by a chemical analysis method to establish the correlation between the teleost embryo toxicity test and chemical contaminants.

## Patentansprüche

1. Verfahren zum Bestimmen einer Gesamttoxizität in einem verzehrbaren Produkt, wobei das Verfahren Folgendes umfasst:
a) Kombinieren eines polaren organischen Lösungsmittels und des verzehrbaren Produkts, um einen polaren organischen Extrakt mit einer Gesamttoxizität zu erhalten;
b) Inkontaktbringen eines Teleost-Embryos mit dem organischen Extrakt aus a); und
c) Bestimmen, ob der Extrakt eine toxische Wirkung auf den Embryo ausübt; wobei eine Toxizitätswirkung auf den Embryo eine Gesamttoxizität des verzehrbaren Produkts anzeigt;
wobei das verzehrbare Produkt ein lipidhaltiges verzehrbares Produkt ist und der polare organische Extrakt aus Schritt a) ferner mit einem unpolaren Lösungsmittel delipidiert wird;
wobei, wenn die Probe eine wenig Lipid enthaltende Probe ist, sie nicht mit einem unpolaren Lösungsmittel delipidiert wird;
wobei das polare organische Lösungsmittel Acetonitril, Ethanol, Propanol, Isopropanol oder ein Gemisch ist, das zwei oder mehr dieser Lösungsmittel enthält;
wobei der Teleost ein Fisch der Gattung Oryzias, der Gattung Dania oder der Gattung Pimephales ist; und
wobei das Volumen des polaren organischen Lösungsmittels, das mit der verzehrbaren Probe kombiniert wird, um ein Gemisch zu bilden, das 1 bis 5-fache des Volumens der Probe beträgt, wenn die Probe eine Flüssigkeit ist; oder das Volumen des polaren organischen Lösungsmittels, das mit der verzehrbaren Probe kombiniert wird, um ein Gemisch zu bilden, das 1 bis 5 fache des Gewichts der Probe beträgt, wenn die Probe ein Feststoff oder Halbfeststoff ist.

2. Verfahren nach Anspruch 1, wobei das verzehrbare Produkt ein Lebensmittel, Speiseöl oder Futtermittel ist, optional wobei das Lebensmittel ein verpacktes Lebensmittel, ein Milchprodukt, Fleisch, Gemüse, Obst, Säuglingsnahrung oder ein Getränk ist.

3. Verfahren nach Anspruch 1, wobei das polare organische Lösungsmittel Acetonitril ist.

4. Verfahren nach einem der vorhergehenden Ansprüche, wobei das Volumen des polaren organischen Lösungsmittels, das mit der verzehrbaren Probe kombiniert wird, um ein Gemisch zu bilden, das 1 bis 3-fache des Volumens der Probe beträgt, wenn die Probe eine Flüssigkeit ist; oder das Volumen des polaren organischen Lösungsmittels, das mit der verzehrbaren Probe kombiniert wird, um ein Gemisch zu bilden, das 1 bis 3 fache des Gewichts der Probe beträgt, wenn die Probe ein Feststoff oder Halbfeststoff ist.

5. Verfahren nach einem der vorhergehenden Ansprüche, wobei vor dem obigen Schritt a) dem verzehrbaren Produkt Wasser zugesetzt wird, wenn das Produkt wasserungesättigt ist.

6. Verfahren nach einem der vorangehenden Ansprüche, wobei optional
(i) das lipidhaltige verzehrbare Produkt ein Lipid enthält, das verhindert, dass der polare organische Extrakt vollständig getrocknet wird;
(ii) das lipidhaltige verzehrbare Produkt ein Lipid von mehr als 5% (Gew./Gew.) enthält; oder
(iii) das unpolare Lösungsmittel Pentan, Cyclopentan, Hexan, Cyclohexan, Benzol, Toluol, Chloroform, Diethylether, Dichlormethan oder ein Gemisch ist, das zwei oder mehr dieser Lösungsmittel enthält.

7. Verfahren nach einem der vorhergehenden Ansprüche, wobei in Schritt a) ein erstes Salz und/oder ein Zucker zugegeben wird, um den polaren organischen Extrakt zu erhalten.

8. Verfahren nach Anspruch 7, wobei das erste Salz Natriumchlorid, Magnesiumsulfat, Natriumsulfat, Calciumsulfat, Calciumchlorid, Magnesiumchlorid, Natriumacetat, Ammoniumacetat, wasserfreies Natriumsulfat, wasserfreies Magnesiumsulfat, wasserfreies Natriumsulfat, wasserfreies Calciumsulfat, wasserfreies Calciumchlorid, wasserfreies Calciumsulfat oder eine Kombination davon ist.

9. Verfahren nach Anspruch 7, wobei der Zucker Saccharose ist.

10. Verfahren nach einem der vorhergehenden Ansprüche, wobei das Verfahren ferner einen Schritt der Zugabe eines zweiten Salzes zu dem organischen Lösungsmittelextrakt aus Schritt (a) umfasst, um ein Gemisch zu bilden.

11. Verfahren nach Anspruch 10, wobei das zweite Salz ein wasserfreies Natriumsulfat, wasserfreies Magnesiumsulfat, wasserfreies Calciumsulfat, wasserfreies Calciumchlorid oder eine Kombination davon ist.

12. Verfahren nach einem der vorhergehenden Ansprüche, wobei das Verfahren ferner einen Schritt des Homogenisierens der Probe vor Schritt (a); des Trocknens des organischen Lösungsmittelextrakts und des erneuten Auflösens des organischen Lösungsmittelextrakts in einem zweiten organischen Lösungsmittel; oder des Rückgewinnens des organischen Lösungsmittelextrakts umfasst.

13. Verfahren nach einem der vorhergehenden Ansprüche, wobei der Teleost-Embryo ein Zebrafisch-Embryo oder Medaka-Embryo ist.

14. Verfahren nach einem der vorhergehenden Ansprüche, wobei die chemischen Verunreinigungen des verzehrbaren Produkts durch ein chemisches Analyseverfahren bestimmt werden, um die Korrelation zwischen dem Teleost-Embryo-Toxizitätstest und den chemischen Verunreinigungen herzustellen.

## Revendications

1. Procédé de détermination d'une toxicité globale dans un produit consommable, comprenant :
a) la combinaison d'un solvant organique polaire et du produit consommable pour obtenir un extrait organique polaire présentant une toxicité globale ;
b) mise en contact d'un embryon de téléostéen avec l'extrait organique de a); et
c) la détermination si l'extrait exerce un effet toxique sur l'embryon ;
dans lequel un effet de toxicité sur l'embryon montre une toxicité globale du produit consommable ;
dans lequel le produit consommable est un produit consommable contenant des lipides, et l'extrait organique polaire de l'étape a) est en outre délipidé avec un solvant non polaire ;
dans lequel si l'échantillon est un échantillon contenant une faible teneur en lipides, il n'est pas délipidé avec un solvant non polaire ;
dans lequel le solvant organique polaire est l'acétonitrile, l'éthanol, le propanol, l'isopropanol ou un mélange contenant deux ou plusieurs solvants de ceux-ci ;
dans lequel le téléostéen est un poisson du genre *Oryzias,* du genre *Danio* ou du genre *Pimephales* ; et
dans lequel le volume du solvant organique polaire combiné avec l'échantillon consommable pour former un mélange est de 1 à 5 fois le volume de l'échantillon, lorsque l'échantillon est un liquide ; ou le volume du solvant organique polaire combiné avec l'échantillon consommable pour former un mélange est de 1 à 5 fois le poids de l'échantillon, lorsque l'échantillon est un solide ou semi-solide.

2. Procédé selon la revendication 1, dans lequel le produit consommable est un aliment, une huile comestible ou un aliment pour animaux, éventuellement dans lequel l'aliment est un aliment emballé, un produit laitier, de la viande, des légumes, des fruits, une préparation pour nourrissons ou une boisson.

3. Procédé selon la revendication 1, dans lequel le solvant organique polaire est l'acétonitrile.

4. Procédé selon l'une quelconque des revendications précédentes, dans lequel le volume du solvant organique polaire combiné avec l'échantillon consommable pour former un mélange est de 1 à 3 fois le volume de l'échantillon, lorsque l'échantillon est un liquide ; ou le volume du solvant organique polaire combiné avec l'échantillon consommable pour former un mélange est de 1 à 3 fois le poids de l'échantillon, lorsque l'échantillon est un solide ou semi-solide.

5. Procédé selon l'une quelconque des revendications précédentes, dans lequel avant l'étape a) ci-dessus, de l'eau est ajoutée au produit consommable lorsque le produit est insaturé en eau.

6. Procédé selon l'une quelconque des revendications précédentes, éventuellement dans lequel
(i) le produit consommable contenant des lipides contient un lipide qui empêche l'extrait organique polaire d'être complètement séché ;
(ii) le produit consommable contenant des lipides contient un lipide supérieur à 5 % (p/p) ; ou
(iii) le solvant non polaire est le pentane, le cyclopentane, l'hexane, le cyclohexane, le benzène, le toluène, le chloroforme, l'éther diéthylique, le dichlorométhane ou un mélange contenant deux ou plusieurs de leurs solvants.

7. Procédé selon l'une quelconque des revendications précédentes, dans lequel dans l'étape a), un premier sel et/ou un sucre est ajouté pour obtenir l'extrait organique polaire.

8. Procédé selon la revendication 7, dans lequel le premier sel est le chlorure de sodium, le sulfate de magnésium, le sulfate de sodium, le sulfate de calcium, le chlorure de calcium, le chlorure de magnésium, l'acétate de sodium, l'acétate d'ammonium, le sulfate de sodium anhydre, le sulfate de magnésium anhydre, le sulfate de sodium anhydre, le sulfate de calcium anhydre, le chlorure de calcium anhydre, le sulfate de calcium anhydre ou une combinaison de ceux-ci.

9. Procédé selon la revendication 7, dans lequel le sucre est le saccharose.

10. Procédé selon l'une quelconque des revendications précédentes, dans lequel le procédé comprend en outre une étape d'ajout d'un second sel à l'extrait de solvant organique de l'étape (a) pour former un mélange.

11. Procédé selon la revendication 10, dans lequel le second sel est un sulfate de sodium anhydre, un sulfate de magnésium anhydre, un sulfate de calcium anhydre, un chlorure de calcium anhydre ou une combinaison de ceux-ci.

12. Procédé selon l'une quelconque des revendications précédentes, dans lequel le procédé comprend en outre une étape d'homogénéisation de l'échantillon avant l'étape (a); le séchage de l'extrait de solvant organique et la redissolution de l'extrait de solvant organique dans un second solvant organique ; ou la récupération de l'extrait de solvant organique.

13. Procédé selon l'une quelconque des revendications précédentes, dans lequel l'embryon de téléostéen est un embryon de poisson zèbre ou un embryon de medaka.

14. Procédé selon l'une quelconque des revendications précédentes, dans lequel les contaminants chimiques du produit consommable sont déterminés par un procédé d'analyse chimique pour établir la corrélation entre le test de toxicité sur les embryons de téléostéens et les contaminants chimiques.
